# EUROPEAN PATENT APPLICATION

(11) **EP 4 393 544 A2**
(43) Date of publication of application: **03.07.2024**
(21) Application number: 23220414.9
(22) Date of filing: 27.12.2023
(51) Int. Cl.: A61N 5/10

(54) **METHOD OF HANDLING RADIATION, AND RADIATION SYSTEM**

(30) Priority: 29.12.2022 SE 2251588
(71) Applicant: Varex Imaging Sweden AB, 182 33 Danderyd (SE)
(72) Inventor: ULLBERG, Christer, 191 34 SOLLENTUNA (SE)
(74) Representative: Kransell & Wennborg KB

(57) **Abstract**

A method of handling radiation in relation to an object (22) comprising providing a primary radiation source (12, 12a), a secondary radiation source (14; 14b) and a primary detector (16; 16a); emitting, by the primary radiation source, primary radiation (20, 20a) onto the object; detecting, by the primary detector, the primary radiation each time during a plurality of primary active periods (76; 76a), the primary radiation having interacted with the object; and emitting, by the secondary radiation source, secondary radiation (24; 24b) onto the object in secondary pulses (80; 80b) emitted during a plurality of primary inactive periods (78; 78a), interleaving each primary inactive period with a primary active period; wherein no secondary pulses are emitted during the primary active periods; and wherein during each primary inactive period, the primary detector is inactive in relation to capturing incoming radiation during this primary inactive period.

## Description

### BACKGROUND

In a radiation system, a radiation source may transmit radiation through an object, such as a patient, and a detector may measure the attenuated radiation. The radiation can be converted to electrical signals, a control system can process these signals and the desired images can be provided.

In some radiation systems, a primary radiation source and a secondary radiation source are used on the same object. Primary radiation from the primary radiation source may then be detected by a primary detector. Secondary radiation from the secondary radiation source may or may not be detected by a secondary detector. One example of such radiation system uses the primary radiation for imaging a patient and the secondary radiation for treating the patient, such as in radiation therapy or radiotherapy.

### BRIEF DESCRIPTION OF SEVERAL VIEWS OF THE DRAWINGS

Further details, advantages and aspects of the present disclosure will become apparent from the following description taken in conjunction with the drawings, wherein:
- FIG. 1:: schematically represents a radiation system according to one example comprising a primary detector;
- FIG. 2:: schematically represents a top view of the primary detector;
- FIG. 3:: schematically represents a partial cross-sectional side view of the primary detector in section A-A in FIG. 2;
- FIG. 4:: schematically represents one example of a readout circuit of the primary detector;
- FIG. 5:: schematically represents a timing diagram of the radiation system;
- FIG. 6:: schematically represents a radiation system according to a further example comprising a primary detector and a secondary detector;
- FIG. 7:: schematically represents a timing diagram of the radiation system in FIG. 6;
- FIG. 8:: is a flowchart outlining general steps of a method.

### DETAILED DESCRIPTION

In the following, a method of handling radiation in relation to an object, and a radiation system for handling radiation in relation to an object, will be described. The same or similar reference numerals will be used to denote the same or similar structural features.

In a radiation system comprising a primary radiation source emitting primary radiation onto an object, a secondary radiation source emitting pulsed secondary radiation onto the object, and a primary detector for detecting the primary radiation having interacted with the object, there may be a risk that scatter from the interaction between the pulsed secondary radiation and the object is recorded by the primary detector. This risk may be reduced by providing an anti-scatter grid on the primary detector. Anti-scatter grids may however provide unsatisfactory performance in that only some of the scatter is blocked, in particular when the secondary radiation has high energies. Moreover, anti-scatter grids add costs and may also deteriorate detection of the primary radiation.

Primary radiation can be emitted onto an object and detected by a primary detector each time during a plurality of primary active periods. Secondary radiation can be emitted onto the object in pulses during a plurality of primary inactive periods but not during the primary active periods. By inactivating the primary detector during each primary inactive period in relation to capturing incoming radiation during this primary inactive period, a risk that scatter from the secondary radiation is recorded by the primary detector is reduced or eliminated without using an anti-scatter grid. Imaging performance of a radiation system can thereby be improved in a cost-efficient manner.

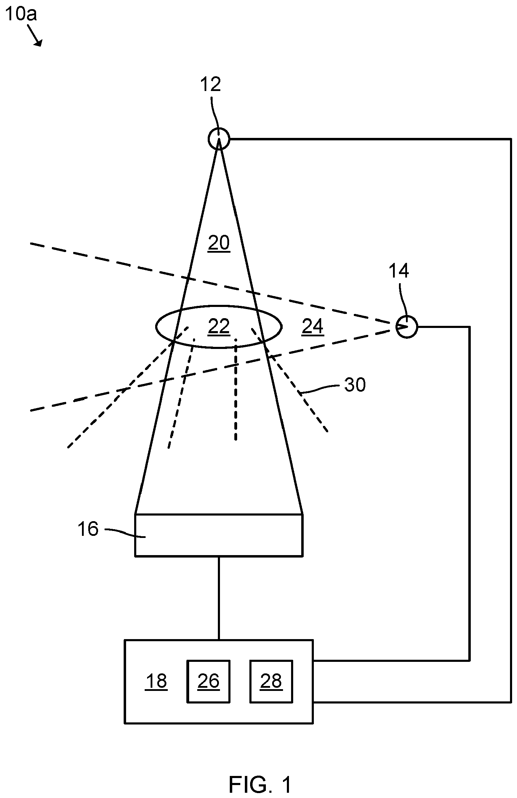

FIG. 1 schematically represents a radiation system 10a according to one example. The radiation system 10a comprises a primary radiation source 12, a secondary radiation source 14, a primary detector 16 and a control system 18. The primary radiation source 12, the primary detector 16 and the control system 18 may be used for computed tomography (CT) scanning.

The primary radiation source 12 is configured to emit primary radiation 20 through an object, here exemplified as a human patient 22. The primary radiation source 12 may for example be an X-ray tube and the primary radiation 20 may for example be X-rays. Moreover, the primary radiation 20 may for example have energies of at least 10 keV (kiloelectronvolt) and/or less than 450 keV. The primary radiation 20 may therefore be referred to as a keV imaging beam. The primary radiation 20 may be emitted in a cone beam. In some examples, the control system 18 controls the primary radiation source 12 to emit the primary radiation 20 in pulses.

After transmission through the patient 22, the primary radiation 20 reaches the primary detector 16 where the primary radiation 20 is detected and directly converted into signals representing a spatially resolved projection image of the patient 22. Thus, by detecting the primary radiation 20 having passed through the patient 22 by the primary detector 16, the patient 22 can be imaged.

The secondary radiation source 14 is configured to emit secondary radiation 24 through the patient 22. The secondary radiation source 14 may for example be a linear accelerator for generating the secondary radiation 24. The secondary radiation 24 may comprise X-rays or electrons. Moreover, the secondary radiation 24 may have energies of at least 0.5 MeV (megaelectronvolt), such as 1 MeV to 10 MeV, such as 2 MeV to 9 MeV. The secondary radiation 24 may therefore be referred to as a MeV treatment beam. The secondary radiation 24 may be used to treat the patient 22, for example against cancer. The secondary radiation 24 may be focused to a treatment area of the patient 22, such as a to a small area containing cancer. In this example, the primary radiation 20 and the secondary radiation 24 are emitted onto the patient 22 in a common plane, here parallel with the drawing plane of FIG. 1.

In an example, the primary radiation source 12 and the secondary radiation source 14 may be arranged to rotate in common around the patient 22. The primary radiation source 12 and the secondary radiation source 14 may thus be fixed with respect to each other. The primary radiation source 12 and the secondary radiation source 14 may for example be spaced with an angular spacing of 90 degrees with respect to a rotation axis centered in the patient 22.

The control system 18 is operatively connected to the primary radiation source 12, the secondary radiation source 14 and the primary detector 16. In FIG. 1, the control system 18 controls the primary radiation source 12 to continuously emit the primary radiation 20. Moreover, the control system 18 controls the secondary radiation source 14 to emit the secondary radiation 24 in pulses, as indicated by the dashed lines of the secondary radiation 24.

The control system 18 is configured to receive imaging data from the primary detector 16, for example in the form of serial data from each readout circuit associated with a respective pixel of the primary detector 16. The control system 18 may be configured to generate two-dimensional (2D) projection images based on the imaging data. The 2D images may be used by the control system 18 to reconstruct, for example three-dimensional (3D) images, of the patient 22 using inter alia known principles of computed tomography.

The control system 18 of this example comprises a data processing device 26 and a memory 28. The memory 28 has a computer program stored thereon. The computer program comprises program code which, when executed by the data processing device 26, causes the data processing device 26 to perform, or command performance of, various steps described herein.

As illustrated in FIG. 1, scatter 30 is generated by the interaction between the primary radiation 20 and the patient 22, and by the interaction between the secondary radiation 24 and the patient 22. The scattering may occur due to absorption inside the patient 22 and the scatter 30 may leave the patient 22 in any angle. A majority of the scatter 30 may be Compton scatter. When the secondary radiation 24 has energies of at least 0.5 MeV, the amount of scatter 30 induced by the secondary radiation 24 may be significant. As illustrated in FIG. 1, part of the scatter 30 from the secondary radiation 24 is incident on the primary detector 16. If the scatter 30 from the secondary radiation 24 is not managed and/or attenuated, the scatter 30 may cause noise in the imaging data of the patient 22 when imaging the primary radiation 20. This noise may cause deterioration of the imaging.

As an alternative to using an anti-scatter grid as mentioned above, effects of the scatter 30 on the imaging can be avoided by first performing and completing a treatment sequence with the secondary radiation 24 and then performing an imaging sequence with the primary radiation 20 (or vice versa). This may however not be desired. For example, it may be desired to provide images as close in time as possible to the pulses of the secondary radiation 24. Moreover, there is a risk that organs of the patient 22 move between such sequences.

The primary detector 16 can be a direct conversion detector or an indirect conversion detector. A direct conversion detector may have advantages over an indirect conversion detector with a scintillator that may experience a residual afterglow effect from the secondary radiation 24, especially with its higher energies, even after the secondary radiation 24 is pulsed off. Thus, in contrast to an indirect conversion detector, such as a scintillator detector, where there is afterglow in the detector, the direct conversion primary detector 16 can be gated to only record radiation during a defined longer time that is closer to the secondary radiation 24 pulse, as described below. Due to these and other advantages of the direct conversion detector, references to the primary detector 16 will refer to features of the direct conversion detector, but the operation may also apply to the indirect conversion detector.

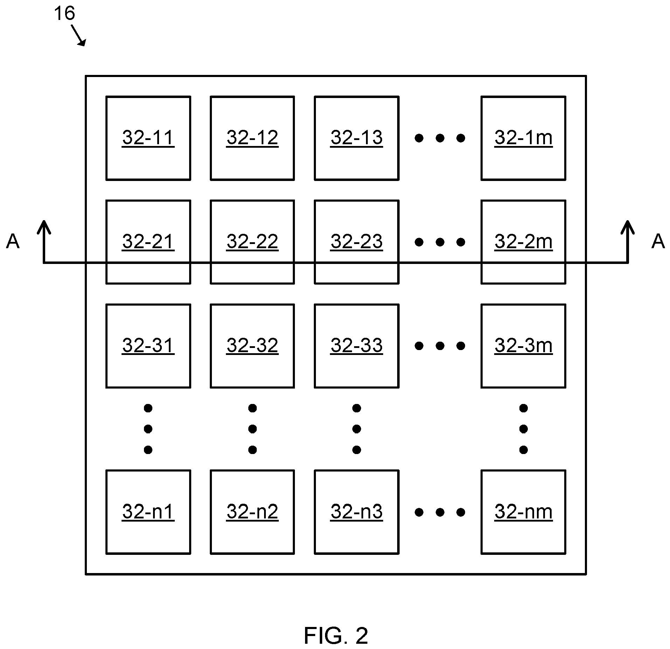

FIG. 2 schematically represents a top view of the primary detector 16. The primary detector 16 comprises a plurality of pixels 32-11 to 32-nm. The primary detector 16 may for example comprise at least 1000 pixels 32-11 to 32-nm. Each pixel 32-11 to 32-nm may also be referred to with reference numeral "32". The pixels 32 may be distributed over at least a major part of the primary detector 16, such as over the entire primary detector 16. In this example, the pixels 32 form a two-dimensional array. The primary detector 16 comprises n rows of pixels 32 and m columns of pixels 32, where each of n and m is a positive integer.

In this example, each pixel 32 has a square shape. Examples of alternative shapes of the pixels 32 comprise non-square rectangular shapes and hexagonal shapes.

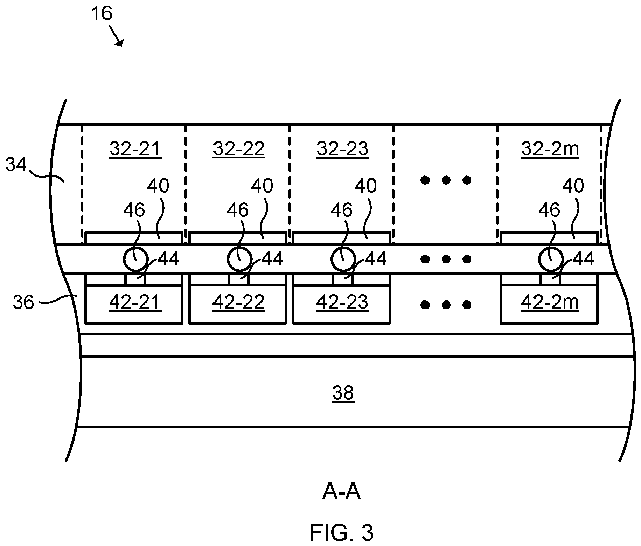

FIG. 3 schematically represents a partial cross-sectional side view of the primary detector 16 in section A-A in FIG. 2. The primary detector 16 of this example comprises a conversion element 34 and a readout substrate 36, for example a readout application-specific integrated circuit (ASIC) substrate. The primary detector 16 of this example further comprises a support substrate 38. The pixels 32 are provided in the conversion element 34.

The conversion element 34 may be constituted by at least one semiconductor substrate, such as a cadmium telluride (CdTe) or cadmium zinc telluride (CdZnTe or CZT) substrate. The conversion element 34 may comprise a continuous conversion substrate or several discrete conversion portions.

The conversion element 34 of this example further comprises a plurality of charge collection electrodes 40, here implemented as contact pads. Each pixel 32 may be defined by a charge collection electrode 40.

When X-rays (or other type of ionizing radiation) impinges on the conversion element 34, electron-hole pairs are created inside the conversion element 34 (thus the term "direct conversion") in response to the absorbed energy. Under the influence of an electric field applied across the conversion element 34, these electrons (holes) are transferred to associated charge collection electrodes 40. Thus, the conversion element 34 is configured to produce one or more charge carriers in response to incident radiation. For example, the conversion element 34 can receive and directly convert incident X-ray photons into electrical charges. According to one example, the primary detector 16 can be inactivated in relation to capturing incoming radiation by inactivating this electric field applied across the conversion element 34.

The readout substrate 36 may comprise a plurality of readout circuits 42-21, 42-22, and 42-23 to 42-2m. Each readout circuit 42-21, 42-22, and 42-23 to 42-2m may also be referred to with reference numeral "42". Each readout circuit 42 comprises a readout electrode 44, here implemented as a contact pad. The primary detector 16 of this example comprises one readout circuit 42 associated with each pixel 32.

The primary detector 16 further comprises a plurality of interconnections 46. Each pair of one pixel 32 and one readout circuit 42 is connected by an interconnection 46. In FIG. 2, the interconnections 46 are exemplified as solder bumps between the charge collection electrodes 40 and the associated readout electrodes 44. Each readout electrode 44 thereby acts as the input to the associated readout circuit 42. Other types of interconnections 46 are however conceivable.

Each readout circuit 42 comprises electronics with functions specific for the associated pixel 32. The readout circuits 42 are arranged to process signals generated by the radiation incident on the conversion element 34.

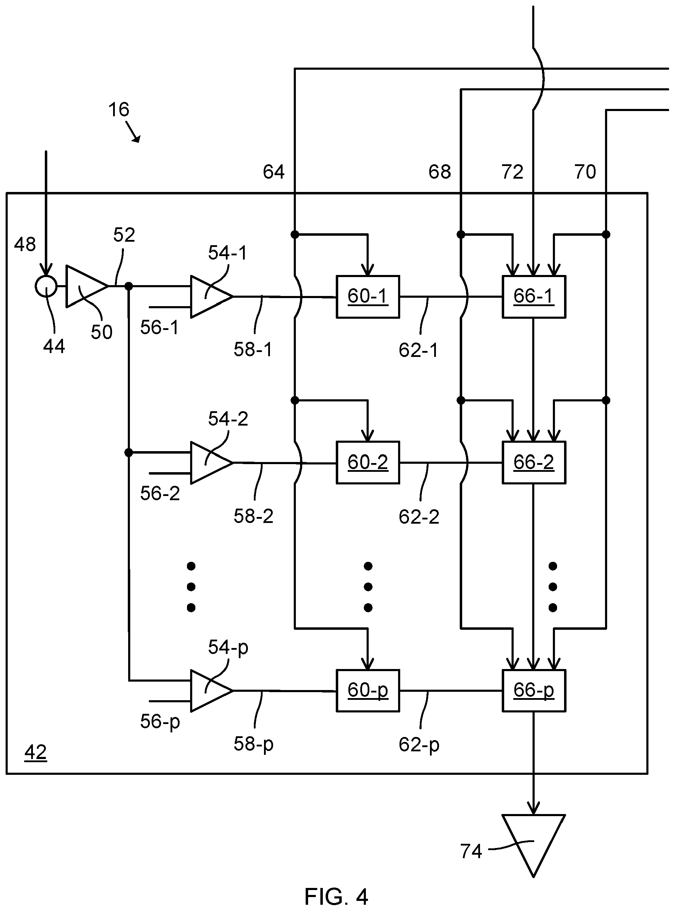

FIG. 4 schematically represents one example of a readout circuit 42 of the primary detector 16. As illustrated, the readout electrode 44 receives an electrical input signal 48 from the associated pixel 32. The primary detector 16 may for example comprise at least 1000 pixels 32 and a readout circuit 42 according to FIG. 4 associated with each pixel 32. The readout circuit 42 is configured to handle the input signals 48 from the associated pixel 32.

The readout circuit 42 of this example comprises a charge sensitive amplifier 50. The charge sensitive amplifier 50 is configured to receive and amplify the input signal 48 from the pixel 32 associated with the readout circuit 42 and to output an electrical amplified signal 52.

The readout circuit 42 of this example further comprises at least one comparator 54. The readout circuit 42 may comprise a plurality of comparators, such as p comparators 54-1 and 54-2 to 54-p, where p is a positive integer. Each comparator 54-1 and 54-2 to 54-p may be referred to with reference numeral "54". For example, an output of the charge sensitive amplifier 50 may be coupled to an input of each comparator 54.

Each comparator 54 comprises a unique threshold value 56-1 and 56-2 to 56-p. FIG. 4 shows p threshold values 56-1 and 56-2 to 56-p. For example, a first comparator 54-1 comprises a first threshold value 56-1 and a second comparator 54-2 comprises a second threshold value 56-2, different from the first threshold value 56-1. Each threshold value 56-1 and 56-2 to 56-p may also be referred to with reference numeral "56".

Each comparator 54 is configured to compare the amplified signal 52 with an associated threshold value 56 and to output a respective detection signal containing respective detection data 58-1 and 58-2 to 58-p if the amplified signal 52 is larger than or smaller than the associated threshold value 56-1 and 56-1 to 56-n. FIG. 4 shows p detection data 58-1 and 58-2 to 58-p. For example, the first comparator 54-1 is configured to compare the amplified signal 52 with the first threshold value 56-1 and to output a first detection signal containing first detection data 58-1 if the amplified signal 52 is larger than the first threshold value 56-1. Correspondingly, the second comparator 54-2 is configured to compare the amplified signal 52 with the second threshold value 56-2 and to output a second detection signal containing second detection data 58-2 if the amplified signal 52 is larger than the second threshold value 56-2. Each detection data 58-1 and 58-2 to 58-p may also be referred to with reference numeral "58". Each detection data 58 may be constituted by a pulse of the respective detection signal. The detection data 58 is thus indicative of the radiation incident on the respective pixel 32 of the primary detector 16.

The readout circuit 42 of this example further comprises one counter 60 associated with each comparator 54. FIG. 4 shows p counters 60-1 and 60-2 to 60-p associated with a common pixel 32. Each counter 60-1 and 60-2 to 60-p may also be referred to with reference numeral "60". For example, a first counter 60-1 may be associated with the first comparator 54-1 and a second counter 60-2 may be associated with the second comparator 54-2. An output of each comparator 54 may be coupled, either directly or indirectly, to an input of the counter 60.

Each counter 60 is configured to count the respective detection data 58, such as pulses of the respective detection signals, to provide respective counted detection data 62. FIG. 4 shows p counted detection data 62-1 and 62-2 to 62-p. Each counted detection data 62-1 and 62-2 to 62-p may also be referred to with reference numeral "62". For example, a first counter 60-1 may count first detection data 58-1 constituted by one or more pulses of a first detection signal as first counted detection data 62-1, and a second counter 60-2 may count second detection data 58-2 constituted by one or more pulses of a second detection signal as second counted detection data 62-2. Each counter 60 may store the respective counted detection data 62 as a counted detection value that is incremented by one for each pulse of the associated detection signal.

Each counter 60 is here configured to be reset in response to a reset signal 64. For example, each of the first counter 60-1 and the second counter 60-2 may be reset in response to the reset signal 64. Each counter 60 may be configured to reset the associated counted detection data 62, for example by resetting the counted detection value to zero, in response to the reset signal 64. The control system 18 may command issuance of the reset signal 64. For example, an output of the control system 18 may be coupled to an input of each counter 60.

The readout circuit 42 of this example further comprises one register 66 associated with each counter 60. FIG. 4 shows p registers 66-1 and 66-2 to 66-p. Each register 66-1 and 66-2 to 66-p may also be referred to with reference numeral "66". For example, an output of each counter 60 may be coupled to an input of the register 66.

Each register 66 is configured to collect respective counted detection data 62 from the associated counter 60 in response to a collection signal 68. For example, a first register 66-1 may collect the first counted detection data 62-1 from the first counter 60-1 in response to the collection signal 68, and a second register 66-2 may collect the second counted detection data 62-2 from the second counter 60-2 in response to the collection signal 68. The control system 18 may command issuance of the collection signal 68. For example, a collection output of the control system 18 may be coupled to a collection input of each register 66.

Each register 66 is configured to read out respective counted detection data 62 in response to a readout signal 70. For example, the first register 66-1 may read out the first counted detection data 62-1 in response to the readout signal 70, and the second register 66-2 may read out the second counted detection data 62-1 in response to the readout signal 70. The control system 18 may command issuance of the readout signal 70. For example, a readout output of the control system 18 may be coupled to a readout input of each register 66.

FIG. 4 further shows a signal line 72. The readout circuit 42 of this example is configured to receive serial data from one or more other readout circuits 42 of the primary detector 16 via the signal line 72. In response to the readout signal 70, each register 66 reads out the respective counted detection data 62 such that the counted detection data 62 is added to the serial data and passed on to the next readout circuit 42 and so on until serial data 74 from a last readout circuit 42 is read out. The serial data 74 may constitute imaging data for processing by the control system 18.

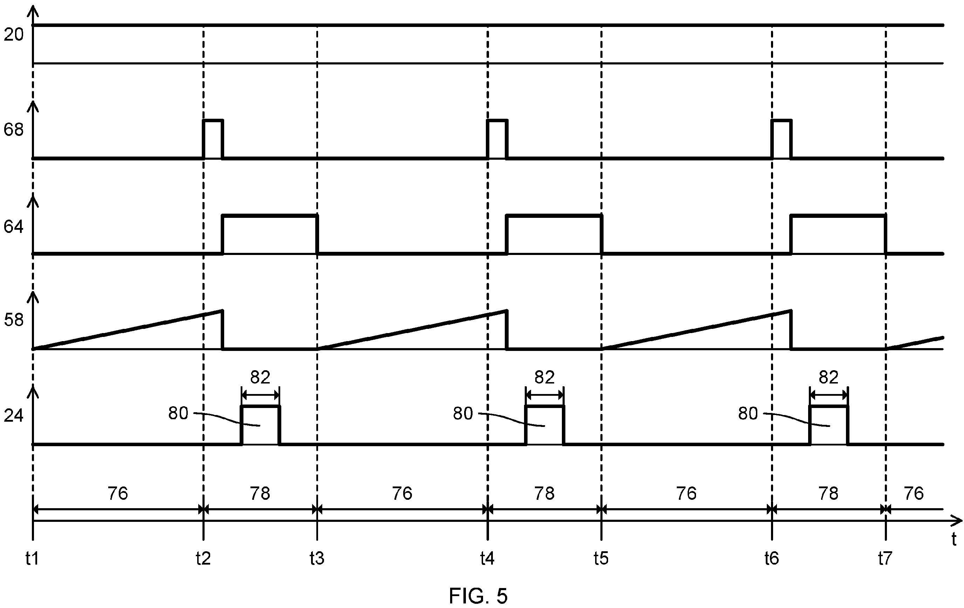

FIG. 5 schematically represents a timing diagram of the radiation system 10a. More specifically, FIG. 5 shows the primary radiation 20, the collection signal 68, the reset signal 64, the detection data 58 and the secondary radiation 24 as a function of time t. FIG. 5 shows a plurality of successive time points t1 to t7. The timing diagram in FIG. 5 is described in connection with a single pixel 32 of the primary detector 16, a single comparator 54, a single counter 60 and a single register 66. However, the timing diagram in FIG. 5 applies correspondingly to each pixel 32 of the primary detector 16 and when using a plurality of comparators 54, counters 60 and registers 66 in each readout circuit 42 associated with each pixel 32.

FIG. 5 shows a plurality of primary active periods 76 and a plurality of primary inactive periods 78. The primary active periods 76 and the primary inactive periods 78 are here alternatingly arranged. Each primary inactive period 78 is interleaved with a primary active period 76. In this example, each primary inactive period 78 is constituted by a time period from an end of a previous primary active period 76 to a start of next primary active period 76, and each primary active period 76 is constituted by a time period from an end of a previous primary inactive period 78 to a start of a next primary inactive period 78. A primary active period 76 here occurs from a first time point t1 to a second time point t2, from a third time point t3 to a fourth time point t4, and from a fifth time point t5 to a sixth time point t6 etc. A primary inactive period 78 here occurs from the second time point t2 to the third time point t3, from the fourth time point t4 to the fifth time point t5, and from the sixth time point t6 to a seventh time point t7 etc.

In this example, the primary radiation 20 is continuously emitted by the primary radiation source 12 and the secondary radiation 24 is emitted in secondary pulses 80. Each secondary pulse 80 is emitted during a primary inactive period 78. Each secondary pulse 80 has a secondary pulse duration 82. In this example, each secondary pulse duration 82 is shorter than the associated primary inactive period 78. Thus, in this example, each primary inactive period 78 contains periods when the secondary pulse 80 is not emitted.

Each secondary pulse duration 82 may be a few µs (microseconds), such as at least 1 µs and/or less than 50 µs. A primary inactive period duration of each primary inactive period 78 may be larger than the associated secondary pulse duration 82 and for example less than 100 µs. The secondary pulses 80 may be repeated every few ms (milliseconds). For example, a time between each two subsequent secondary pulses 80 may be 100 ms, such as at least 1 ms and/or less than 500 ms. Thus, a primary active period duration of each primary active period 76 may be at least 1 ms. In view of this, it is apparent that the timing diagram in FIG. 5 may not be to scale.

In this example, no collection signal 68 is sent to the register 66 and no reset signal 64 is sent to the counter 60 during the primary active periods 76. As a consequence, the detection data 58 of the counter 60 is incrementally increased due to primary radiation 20 during each primary active period 76 and the counter 60 holds corresponding counted detection data 62 until receiving the reset signal 64 in the next primary inactive period 78. The primary detector 16 is thus configured to detect the primary radiation 20 each time during a plurality of primary active periods 76.

In this example, the collection signal 68 is sent to the register 66 in each primary inactive period 78. The register 66 thereby collects the counted detection data 62 from the counter 60 in each primary inactive period 78. After the collection signal 68 is sent to the register 66, the reset signal 64 is sent to the counter 60. As shown in FIG. 5, the reset signal 64 is here issued continuously during the remainder of each primary inactive period 78, holding the counter 60 in a reset state. In these ways, no detection data 58 is collected during the primary inactive periods 78. The counter 60 is thus reset during each primary inactive period 78.

As shown in FIG. 5, the detection data 58 is also incrementally increased initially during each primary inactive period 78. However, since the collection signal 68 is here sent immediately at the start of each primary inactive period 78, counted detection data 62 corresponding to this additional detection data 58 is not collected by the register 66. Thus, by sending the collection signal 68 immediately at the start of each primary inactive period 78, and by issuing the reset signal 64 at the end of each primary inactive period 78, it can be ensured that the counted detection data 62 only corresponds to detection data 58 from the respective previous primary active period 76. Moreover, the sending of the collection signal 68 immediately at the start of each primary inactive period 78, and the issuance of the reset signal 64 at the end of each primary inactive period 78, constitutes one example of inactivating the primary detector 16 in relation to capturing incoming radiation during the primary inactive periods 78. Thus, in each primary active period 76, the primary detector 16 is in an active state in relation to capturing incoming radiation during this primary active period 76, and in each primary inactive period 78, the primary detector 16 is in an inactive state in relation to capturing incoming radiation during this primary inactive period 78.

Furthermore, since the reset signal 64 is issued at the end of each primary inactive period 78, detection data 58 from incoming radiation during the primary inactive periods 78 is not acquired, here not counted by the counter 60. Moreover, by sending the collection signal 68 and emitting a secondary pulse 80 during each primary inactive period 78, images that are closed in time to the respective secondary pulses 80 can be provided.

In this example, one secondary pulse 80 of secondary radiation 24 is emitted during each primary inactive period 78, and no secondary pulses 80 of secondary radiation 24 are emitted during the primary active periods 76. Since no secondary pulses 80 are emitted during the primary active periods 76 and since the primary detector 16 is inactive in each primary inactive period 78 in relation to capturing incoming radiation during this primary inactive period 78, the primary detector 16 performs a gated acquisition that eliminates recording of any scatter 30 from the secondary radiation 24. This gated acquisition is synchronized with the secondary pulses 80 such that only radiation detected during the primary active periods 76, when no secondary pulses 80 are emitted, is acquired. With this capability of the primary detector 16, the collection of photons can be set to only record photons from the primary detector 16 synchronized while the secondary radiation source 14 does not output a secondary pulse 80. This gated acquisition is cost-efficient since the primary detector 16 does not need to be provided with an anti-scatter grid. Moreover, in some examples, the loss of signal can be very low, such as approximately 0.1% loss, since the acquisition will be off during a few µs every few ms.

The readout signal 70 may be sent to the register 66 at any time to cause the register 66 to read out respective counted detection data 62. For example, the readout signal 70 may be sent during each primary inactive period 78. Alternatively, the readout signal 70 may be sent to the register 66 during only some of the primary inactive periods 78, such as every n'th primary inactive period 78, where n is a positive integer.

Although the radiation system 10a has been exemplified in connection with a particular medical application, the radiation system 10a can be used in any application where detection of primary radiation 20 from a primary radiation source 12 is impaired by scatter 30 from pulsed secondary radiation 24.

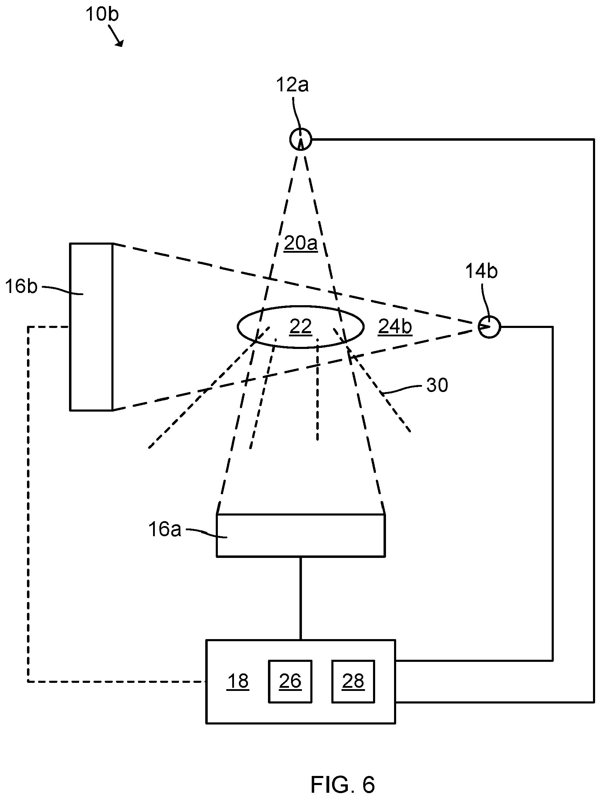

FIG. 6 schematically represents a radiation system 10b according to a further example. Mainly differences with respect to the radiation system 10a will be described. The radiation system 10b comprises a primary radiation source 12a configured to emit primary radiation 20a, a secondary radiation source 14b configured to emit secondary radiation 24b, a primary detector 16a and a secondary detector 16b. The control system 18 is operatively connected to the primary radiation source 12a, the secondary radiation source 14b, the primary detector 16a and the secondary detector 16b. The primary radiation source 12a is here identical to the primary radiation source 12 and the primary detector 16a is here identical to the primary detector 16. In this example, the secondary detector 16b is of the same type as the primary detector 16a, e.g., identical thereto. Also the secondary detector 16b may thus be a direct conversion detector or an indirect conversion detector.

In this example, the secondary radiation source 14b is of the same type as the primary radiation source 12a, e.g., identical thereto. Alternatively, the secondary radiation source 14b may be of the same type as the secondary radiation source 14, e.g., identical thereto. In case the secondary radiation source 14b is an MeV radiation source, like the secondary radiation source 14, the secondary detector 16b may be an MeV detector. In this case, the secondary detector 16b may be a direct conversion detector or an indirect conversion detector.

In this example, the primary radiation 20a and the secondary radiation 24b are emitted onto the patient 22 unique planes. That is, the primary radiation 20a and the secondary radiation 24b are offset in a direction normal to the drawing plane in FIG. 6.

In this example, the control system 18 controls the primary radiation source 12a to emit the primary radiation 20a onto the patient 22 in primary pulses and controls the secondary radiation source 14b to emit the secondary radiation 24b onto the patient 22 in secondary pulses.

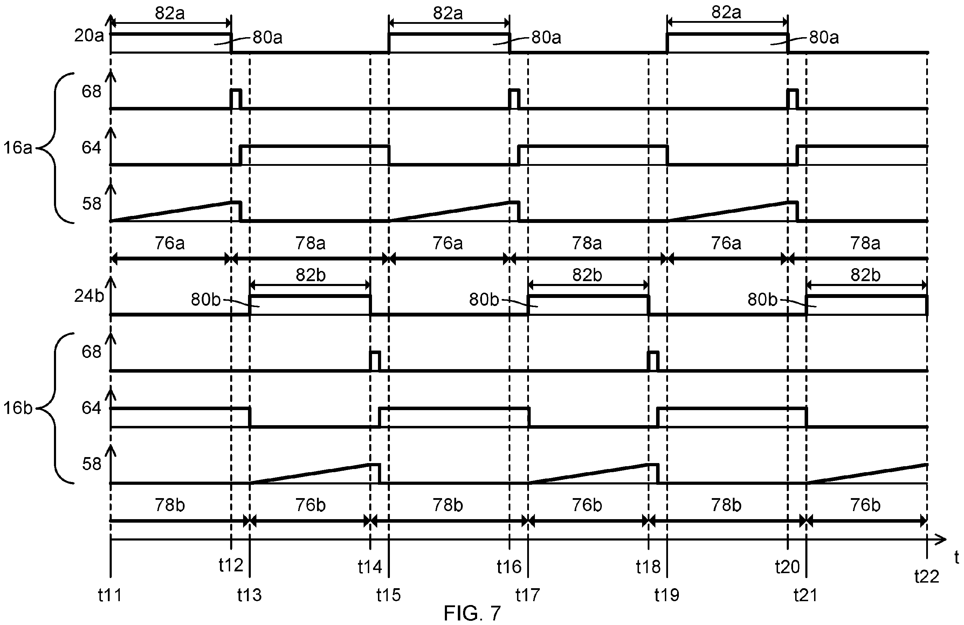

FIG. 7 schematically represents a timing diagram of the radiation system 10b. Mainly differences with respect to FIG. 5 will be described. In FIG. 7, the primary pulses 80a of the primary radiation 20a and the secondary pulses 80b of the secondary radiation 24b are shown as a function of time t. In addition to the collection signal 68, the reset signal 64 and the detection data 58 associated with the primary detector 16a, FIG. 7 also shows a collection signal 68, a reset signal 64 and detection data 58 associated with the secondary detector 16b, which is here exemplified as being of the same type as the primary detector 16a.

FIG. 7 shows a plurality of successive time points t11 to t22. The timing diagram in FIG. 7 is described in connection with a single pixel 32, a single comparator 54, a single counter 60 and a single register 66 of the primary detector 16a, and in connection with a single pixel 32, a single comparator 54, a single counter 60 and a single register 66 of the secondary detector 16b. However, the timing diagram in FIG. 7 applies correspondingly to each pixel 32 of the primary detector 16a and when using a plurality of comparators 54, counters 60 and registers 66 in each readout circuit 42 associated with each pixel 32 of the primary detector 16a and to each pixel 32 of the secondary detector 16b and when using a plurality of comparators 54, counters 60 and registers 66 in each readout circuit 42 associated with each pixel 32 of the secondary detector 16b.

FIG. 7 shows a plurality of primary active periods 76a, a plurality of primary inactive periods 78a, a plurality of secondary active periods 76b and a plurality of secondary inactive periods 78b. The primary active periods 76a and the primary inactive periods 78a may correspond to the primary active periods 76 and the primary inactive periods 78, respectively. Similarly to the primary active periods 76a and the primary inactive periods 78a, also the secondary active periods 76b and the secondary inactive periods 78b are alternatingly arranged. Each secondary inactive period 78b is interleaved with a secondary active period 76b. In this example, each secondary inactive period 78b is constituted by a time period between two secondary active periods 76b. A primary active period 76a here occurs from an eleventh time point t11 to a twelfth time point t12, from a fifteenth time point t15 to a sixteenth time point t16, and from a nineteenth time point t19 to a twentieth time point t20 etc. A primary inactive period 78a here occurs from the twelfth time point t12 to the fifteenth time point t15, and from the sixteenth time point t16 to the nineteenth time point t19 etc. A secondary active period 76b here occurs from a thirteenth time point t13 to a fourteenth time point t14, from a seventeenth time point t17 to an eighteenth time point t18, and from a twenty-first time point t21 to a twenty-second time point t22 etc. A secondary inactive period 78b here occurs from the fourteenth time point t14 to the seventeenth time point t17, and from the eighteenth time point t18 to the twenty-first time point t21 etc.

In this example, one primary pulse 80a is emitted during each secondary inactive period 78b and one secondary pulse 80b is emitted during each primary inactive period 78a. Although not shown, primary pulses 80a may be emitted during a ramp-up at the end of the secondary active period 76b and extend into the beginning of the secondary active period 76b but no secondary pulses 80b are emitted during the primary active periods 76a. Moreover, in this example, each primary pulse 80a is emitted primarily during a primary active period 76a and each secondary pulse 80b is emitted during a secondary active period 76b. The primary active periods 76a here occur during the secondary inactive periods 78b and are shorter than the secondary inactive periods 78b. Correspondingly, the secondary active periods 76b here occur during the primary inactive periods 78a and are shorter than the primary inactive periods 78a. Each primary pulse 80a has a primary pulse duration 82a, and each secondary pulse 80b has a secondary pulse duration 82b.

Each primary pulse duration 82a may or may not be longer than an associated secondary inactive period 78b. For example, with respect to each primary active period 76a, the associated primary pulse 80a may be ramped up at the end of a preceding primary inactive period 78a (e.g., a setup time) and may be ramped down at the beginning of a succeeding primary inactive period 78a (e.g., a hold time). In any case, each secondary inactive period 78b may contain periods when the primary pulse 80a is not emitted. In this example, each primary pulse duration 82a may be equal to or greater than each primary active period 76a.

Each secondary pulse duration 82b here occurs within, and is shorter than, an associated primary inactive period 78a. Thus, each primary inactive period 78a here contains periods when the secondary pulse 80b is not emitted. In this example, each secondary pulse duration 82b is equal to each secondary active period 76b.

One or both of the primary pulse duration 82a and the secondary pulse duration 82b may be a few µs, such as at least 1 µs and/or less than 100 µs. Alternatively, one of the primary pulse duration 82a and the secondary pulse duration 82b may be longer than the other of the primary pulse duration 82a and the secondary pulse duration 82b, such as at least 50 % longer, such as at least 100 % longer, such as at least 1 ms and/or less than 10 ms. Alternatively, both of the primary pulse duration 82a and the secondary pulse duration 82b may be a few ms, such as at least 1 ms and/or less than 10 ms. In any case, each primary pulse duration 82a may be at least 90 % of the associated secondary inactive period 78b and/or each secondary pulse duration 82b may be at least 90 % of the associated primary inactive period 78a.

In this example, no collection signal 68 is sent to the register 66 of the primary detector 16a and no reset signal 64 is sent to the counter 60 of the primary detector 16a during the primary active periods 76a. As a consequence, the detection data 58 of the counter 60 of the primary detector 16a is incrementally increased due to the primary radiation 20a during each primary active period 76a and the counter 60 of the primary detector 16a holds corresponding counted detection data 62 until receiving the reset signal 64 in the next primary inactive period 78a. The primary detector 16a is thus configured to detect the primary radiation 20a each time during a plurality of primary active periods 76a.

In this example, the collection signal 68 is sent to the register 66 of the primary detector 16a in each primary inactive period 78a. The register 66 thereby collects the counted detection data 62 from the counter 60 in each primary inactive period 78a. After the collection signal 68 is sent to the register 66, the reset signal 64 is sent to the counter 60 of the primary detector 16a. As shown in FIG. 7, the reset signal 64 is here issued continuously during the remainder of each primary inactive period 78a, holding the counter 60 of the primary detector 16a in a reset state. The counter 60 of the primary detector 16a is thereby being reset in each primary inactive period 78a. Each primary inactive period 78a may thus be defined by the associated collection signal 68 and the associated reset signal 64.

By sending the collection signal 68 to the register 66 of the primary detector 16a immediately at the start of each primary inactive period 78a, and by issuing the reset signal 64 at the end of each primary inactive period 78a, it can be ensured that the counted detection data 62 in the primary detector 16a only corresponds to detection data 58 from the respective previous primary active period 76a. Thus, in each primary active period 76a, the primary detector 16a is in an active state in relation to capturing incoming radiation during this primary active period 76a, and in each primary inactive period 78a, the primary detector 16a is in an inactive state in relation to capturing incoming radiation during this primary inactive period 78a.

Furthermore, since the reset signal 64 is sent to the counter 60 of the primary detector 16a at the end of each primary inactive period 78a, detection data 58 from incoming radiation during the primary inactive periods 78a is not acquired by the primary detector 16a. The readout signal 70 may for example be sent to the register 66 of the primary detector 16a during each primary inactive period 78a.

In this example, no collection signal 68 is sent to the register 66 of the secondary detector 16b and no reset signal 64 is sent to the counter 60 of the secondary detector 16b during the secondary active periods 76b. As a consequence, the detection data 58 of the counter 60 of the secondary detector 16b is incrementally increased due to primary radiation 20a during each secondary active period 76b and the counter 60 of the secondary detector 16b holds corresponding counted detection data 62 until receiving the reset signal 64 in the next secondary inactive period 78b. The secondary detector 16b is thus configured to detect the secondary radiation 24b each time during a plurality of secondary active periods 76b.

In this example, the collection signal 68 is sent to the register 66 of the secondary detector 16b in each secondary inactive period 78b. The register 66 thereby collects the counted detection data 62 from the counter 60 in each secondary inactive period 78b. After the collection signal 68 is sent to the register 66, the reset signal 64 is sent to the counter 60 of the secondary detector 16b. As shown in FIG. 7, the reset signal 64 is here issued continuously during the remainder of each secondary inactive period 78b, holding the counter 60 of the secondary detector 16b in a reset state. The counter 60 of the secondary detector 16b is thus being reset in each secondary inactive period 78b.

By sending the collection signal 68 to the register 66 of the secondary detector 16b immediately at the start of each secondary inactive period 78b, and by issuing the reset signal 64 at the end of each secondary inactive period 78b, it can be ensured that the counted detection data 62 in the secondary detector 16b only corresponds to detection data 58 from the respective previous secondary active period 76b. Thus, in each secondary active period 76b, the secondary detector 16b is in an active state in relation to capturing incoming radiation during this secondary active period 76b, and in each secondary inactive period 78b, the secondary detector 16b is in an inactive state in relation to capturing incoming radiation during this secondary inactive period 78b.

Furthermore, since the reset signal 64 is sent to the counter 60 of the secondary detector 16b at the end of each secondary inactive period 78b, detection data 58 from incoming radiation during the secondary inactive periods 78b is not acquired by the secondary detector 16b. The readout signal 70 may for example be sent to the register 66 of the secondary detector 16b during each secondary inactive period 78b.

The radiation system 10b enables primary images from a primary set of a primary radiation source 12a and a primary detector 16a to be obtained close in time to secondary images obtained from a secondary set of a secondary radiation source 14b and a secondary detector 16b, without the primary images being corrupted or distorted by scatter 30 from the secondary radiation 24b and/or without the secondary images being corrupted or distorted by scatter 30 from the primary radiation 20a.

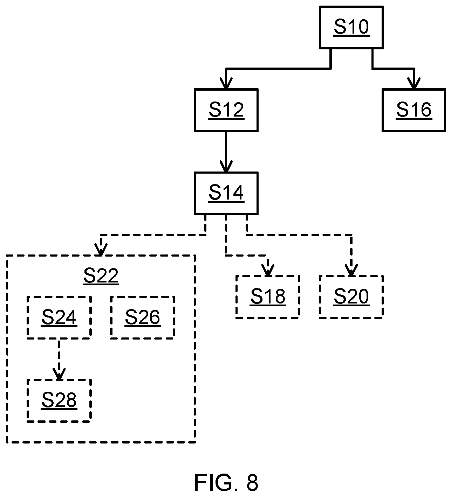

FIG. 8 is a flowchart outlining general steps of a method of handling radiation in relation to an object 22. The method comprises providing S10 a primary radiation source 12, 12a, a secondary radiation source 14, 14b and a primary detector 16, 16a. The method further comprises emitting S12, by the primary radiation source 12, 12a, primary radiation 20, 20a onto the object 22. The method further comprises detecting S14, by the primary detector 16, 16a, the primary radiation 20, 20a each time during a plurality of primary active periods 76, 76a, the primary radiation 20, 20a having interacted with the object 22. The method further comprises emitting S16, by the secondary radiation source 14, 14b, secondary radiation 24, 24b onto the object 22 in secondary pulses 80, 80b emitted during a plurality of primary inactive periods 78, 78a, interleaving each primary inactive period 78, 78a with a primary active period 76, 76a, wherein no secondary pulses 80, 80b are emitted during the primary active periods 76, 76a, and wherein during each primary inactive period 78, 78a, the primary detector 16, 16a is inactive in relation to capturing incoming radiation during this primary inactive period 78, 78a.

The method of this example further comprises not detecting S18, by the primary detector 16, 16a, incoming radiation during the primary inactive periods 78, 78a. The method of this example further comprises not collecting S20, by the primary detector 16, 16a, detection data 58 indicative of incoming radiation during the primary inactive periods 78, 78a.

The method of this example further comprises collecting S22, by the primary detector 16, 16a, detection data 58 indicative of the primary radiation 20, 20a detected during the primary active periods 76, 76a. The method of this example further comprises collecting S24, by each register 66 and during each primary inactive period 78, 78a, the counted detection data 62 from the associated counter 60. The method of this example further comprises resetting S26 each counter 60 during each primary inactive period 78, 78a. The method of this example further comprises reading out S28 the counted detection data 62 from each register 66 during at least some of the primary inactive periods 78, 78a.

Some embodiments include a method of handling radiation in relation to an object 22, the method comprising providing S10 a primary radiation source 12, 12a, a secondary radiation source 14, 14b and a primary detector 16, 16a; emitting S12, by the primary radiation source 12, 12a, primary radiation 20, 20a onto the object 22; detecting S14, by the primary detector 16, 16a, the primary radiation 20, 20a each time during a plurality of primary active periods 76, 76a, the primary radiation 20, 20a having interacted with the object 22; and emitting S16, by the secondary radiation source 14, 14b, secondary radiation 24, 24b onto the object 22 in secondary pulses 80, 80b emitted during a plurality of primary inactive periods 78, 78a, interleaving each primary inactive period 78, 78a with a primary active period 76, 76a; wherein no secondary pulses 80, 80b are emitted during the primary active periods 76, 76a; and wherein during each primary inactive period 78, 78a, the primary detector 16, 16a is inactive in relation to capturing incoming radiation during this primary inactive period 78, 78a.

The secondary pulses 80, 80b are synchronized with the primary inactive periods 78, 78a and are emitted during the primary inactive periods 78, 78a. For example, one secondary pulse 80, 80b may be emitted during each primary inactive periods 78, 78a.

The primary radiation 20, 20a may or may not be emitted in primary pulses 80a. In case the primary radiation 20, 20a is emitted in primary pulses 80a, the primary pulses 80a may occur primarily during the primary active periods 76, 76a.

The secondary radiation source 14, 14b may be separated from the primary radiation source 12, 12a. For example, the secondary radiation source 14, 14b may be angled 5 degrees to 170 degrees, such as 90 degrees, relative to the primary radiation source 12, 12a and with respect to a rotation axis centered in the object 22.

In some embodiments, the primary detector 16, 16a does not detect S18 incoming radiation during the primary inactive periods 78, 78a and/or does not collect S20 detection data 58 indicative of incoming radiation during the primary inactive periods 78, 78a. In these manners, the primary detector 16, 16a is inactive in relation to capturing incoming radiation. When the primary detector 16, 16a does not detect incoming radiation or does not collect detection data 58 indicative of incoming radiation, the incoming radiation is not captured by the primary detector 16, 16a.

In some embodiments, the method further comprises collecting S22, by the primary detector 16, 16a, detection data 58 indicative of the primary radiation 20, 20a detected during the primary active periods 76, 76a. The collection by the primary detector 16, 16a of the detection data 58 indicative of the primary radiation 20, 20a detected during a primary active period 76, 76a may take place during this primary active period 76, 76a or during a subsequent primary inactive period 78, 78a, e.g., before a secondary pulse 80, 80b is emitted in that primary inactive period 78, 78a.

In some embodiments, the primary detector 16, 16a comprises a plurality of pixels 32, one or more counters 60 associated with each pixel 32, each configured to count the detection data 58 indicative of the primary radiation 20, 20a detected during the primary active periods 76, 76a to provide counted detection data 62, and a register 66 associated with each counter 60, each register 66 being configured to collect the counted detection data 62 from the associated counter 60.

In some embodiments, the method further comprises collecting S24, by each register 66 and during each primary inactive period 78, 78a, the counted detection data 62 from the associated counter 60.

In some embodiments, the method further comprises resetting S26 each counter 60 during each primary inactive period 78, 78a.

In some embodiments, the method further comprises reading out S28 the counted detection data 62 from each register 66 during at least some of the primary inactive periods 78, 78a. According to one example the method comprises reading out the counted detection data 62 from each register 66 during each primary inactive period 78, 78a.

In some embodiments, the secondary radiation source 14, 14b is a linear accelerator.

In some embodiments, a secondary pulse duration 82, 82b of each secondary pulse 80, 80b is less than 100 µs. The secondary pulse duration 82, 82b may be shorter than the primary inactive period 78, 78a containing the secondary pulse 80, 80b. In this case, the primary inactive periods 78, 78a may include periods when the secondary radiation source 14, 14b does not generate secondary pulses 80, 80b.

In some embodiments, a primary active period duration of each primary active period 76, 76a is at least 1 ms.

In some embodiments, the secondary radiation 24, 24b has energies of at least 0.5 MeV.

In some embodiments, emitting the primary radiation 20, 20a onto the object 22 occurs in primary pulses 80a by the primary radiation source 12, 12a. The primary pulses 80a may be emitted primarily during the plurality of primary active periods 76, 76a. A primary pulse duration 82a of each primary pulse 80a may be less than 100 µs.

In some embodiments, the primary radiation 20, 20a is detected by the primary detector 16, 16a by direct conversion each time during the plurality of primary active periods 76, 76a. In this case, the primary detector 16, 16a may be a direct conversion detector. Alternatively, the primary radiation 20, 20a may be detected by the primary detector 16, 16a by indirect conversion each time during the plurality of primary active periods 76, 76a. In this case, the primary detector 16, 16a may be an indirect conversion detector.

In some embodiments, the method further comprises detecting, by a secondary detector 16b, the secondary radiation 24b each time during a plurality of secondary active periods 76b, the secondary radiation 24b having interacted with the object 22. The secondary detector 16b may be a direct conversion detector or an indirect conversion detector. In the former case, the detection by the secondary detector 16b may take place during a plurality of secondary active periods 76b. The secondary pulses 80b may be emitted during the secondary active periods 76b. During each of a plurality of secondary inactive periods 78b, the secondary detector 16b may be inactive in relation to capturing incoming radiation during this secondary inactive period 78b. Each secondary inactive period 78b may follow a unique secondary active period 76b.

The primary inactive periods 78a may include the secondary active periods 76b, and the secondary active periods 76b may include the secondary pulses 80b. Correspondingly, the secondary inactive periods 78b may include the primary active periods 76a, and the primary active periods 76a may include the primary pulses 80a.

Some embodiments include a radiation system 10a, 10b for handling radiation in relation to an object 22, the radiation system 10a, 10b comprising a primary radiation source 12, 12a configured to emit primary radiation 20, 20a; a secondary radiation source 14, 14b configured to emit secondary radiation 24, 24b; a primary detector 16, 16a configured to detect the primary radiation 20, 20a each time during a plurality of primary active periods 76, 76a, the primary radiation 20, 20a having interacted with the object 22; a control system 18 comprising at least one data processing device 26 and at least one memory 28 having at least one computer program stored thereon, the at least one computer program comprising program code which, when executed by the at least one data processing device 26, causes the at least one data processing device 26 to control the primary radiation source 12, 12a to emit the primary radiation 20, 20a onto the object 22; control the secondary radiation source 14, 14b to emit the secondary radiation 24, 24b onto the object 22 in secondary pulses 80, 80b emitted during a plurality of primary inactive periods 78, 78a, interleaving each primary inactive period 78, 78a with a primary active period 76, 76a; control the secondary radiation source 14, 14b to not emit any secondary pulses 80, 80b during the primary active periods 76, 76a; and control the primary detector 16, 16a to adopt an inactive state during each primary inactive period 78, 78a, where the primary detector 16, 16a is inactive in relation to capturing incoming radiation during this primary inactive period 78, 78a.

In some embodiments, the at least one computer program comprises program code which, when executed by the at least one data processing device 26, causes the at least one data processing device 26 to control the primary detector 16, 16a to not detect incoming radiation during the primary inactive periods 78, 78a; and/or control the primary detector 16, 16a to not collect detection data 58 indicative of incoming radiation during the primary inactive periods 78, 78a.

In some embodiments, the at least one computer program comprises program code which, when executed by the at least one data processing device 26, causes the at least one data processing device 26 to control the primary detector 16, 16a to collect detection data 58 indicative of the primary radiation 20, 20a detected during the primary active periods 76, 76a.

In some embodiments, the primary detector 16, 16a comprises a plurality of pixels 32, one or more counters 60 associated with each pixel 32, each configured to count the detection data 58 indicative of the primary radiation 20, 20a detected during the primary active periods 76, 76a to provide counted detection data 62, and a register 66 associated with each counter 60, each register 66 being configured to collect the counted detection data 62 from the associated counter 60.

In some embodiments, each register 66 is configured to collect the counted detection data 62 from the associated counter 60 in response to a collection signal 68. In this case, the at least one computer program may comprise program code which, when executed by the at least one data processing device 26, causes the at least one data processing device 26 to command sending of the collection signal 68 to each register 66 during each primary inactive period 78, 78a.

In some embodiments, each counter 60 is configured to be reset in response to a reset signal 64. In this case, the at least one computer program may comprise program code which, when executed by the at least one data processing device 26, causes the at least one data processing device 26 to command sending of the reset signal 64 to each counter 60 during each primary inactive period 78, 78a.

In some embodiments, each register 66 is configured to read out the counted detection data 62 in response to a readout signal 70. In this case, the at least one computer program may comprise program code which, when executed by the at least one data processing device 26, causes the at least one data processing device 26 to command sending of the readout signal 70 to each register 66 during at least some of the primary inactive periods 78, 78a.

In some embodiments, the secondary radiation source 14, 14b is a linear accelerator.

In some embodiments, a secondary pulse duration 82, 82b of each secondary pulse 80, 80b is less than 100 µs.

In some embodiments, a primary active period duration of each primary active period 76, 76a is at least 1 ms.

In some embodiments, the secondary radiation 24, 24b has energies of at least 0.5 MeV.

In some embodiments, the at least one computer program comprises program code which, when executed by the at least one data processing device 26, causes the at least one data processing device 26 to control the primary radiation source 12, 12a to emit the primary radiation 20, 20a onto the object 22 in primary pulses 80a. The primary pulses 80a may be emitted primarily during the plurality of primary active periods 76, 76a.

In some embodiments, the primary detector is a direct conversion detector.

While the present disclosure has been described with reference to exemplary embodiments, it will be appreciated that the present invention is not limited to what has been described above. For example, it will be appreciated that the dimensions of the parts may be varied as needed. Accordingly, it is intended that the present invention may be limited only by the scope of the claims appended hereto.

## Claims

1. A method of handling radiation in relation to an object (22), the method comprising:
- providing (S10) a primary radiation source (12, 12a), a secondary radiation source (14; 14b) and a primary detector (16; 16a);
- emitting (S12), by the primary radiation source (12, 12a), primary radiation (20, 20a) onto the object (22);
- detecting (S14), by the primary detector (16; 16a), the primary radiation (20, 20a) each time during a plurality of primary active periods (76; 76a), the primary radiation (20, 20a) having interacted with the object (22); and
- emitting (S16), by the secondary radiation source (14; 14b), secondary radiation (24; 24b) onto the object (22) in secondary pulses (80; 80b) emitted during a plurality of primary inactive periods (78; 78a), interleaving each primary inactive period (78; 78a) with a primary active period (76; 76a);
wherein no secondary pulses (80; 80b) are emitted during the primary active periods (76; 76a); and
wherein during each primary inactive period (78; 78a), the primary detector (16; 16a) is inactive in relation to capturing incoming radiation during this primary inactive period (78; 78a).

2. The method according to claim 1, wherein the primary detector (16; 16a) does not detect (S18) incoming radiation during the primary inactive periods (78; 78a) and/or does not collect (S20) detection data (58) indicative of incoming radiation during the primary inactive periods (78; 78a).

3. The method according to any of the preceding claims, further comprising collecting (S22), by the primary detector (16; 16a), detection data (58) indicative of the primary radiation (20, 20a) detected during the primary active periods (76; 76a).

4. The method according to claim 3, wherein the primary detector (16; 16a) comprises a plurality of pixels (32), one or more counters (60) associated with each pixel (32), each configured to count the detection data (58) indicative of the primary radiation (20, 20a) detected during the primary active periods (76; 76a) to provide counted detection data (62), and a register (66) associated with each counter (60), each register (66) being configured to collect the counted detection data (62) from the associated counter (60).

5. The method according to claim 4, further comprising collecting (S24), by each register (66) and during each primary inactive period (78; 78a), the counted detection data (62) from the associated counter (60).

6. The method according to claim 4 or 5, further comprising resetting (S26) each counter (60) during each primary inactive period (78; 78a).

7. The method according to any of claims 4 to 6, wherein the method further comprises reading out (S28) the counted detection data (62) from each register (66) during at least some of the primary inactive periods (78; 78a).

8. The method according to any of the preceding claims, wherein the secondary radiation source (14; 14b) is a linear accelerator.

9. The method according to any of the preceding claims, wherein a secondary pulse duration (82, 82b) of each secondary pulse (80; 80b) is less than 100 µs.

10. The method according to any of the preceding claims, wherein a primary active period duration of each primary active period (76; 76a) is at least 1 ms.

11. The method according to any of the preceding claims, wherein the secondary radiation (24; 24b) has energies of at least 0.5 MeV.

12. The method according to any of the preceding claims, wherein emitting the primary radiation (20, 20a) onto the object (22) occurs in primary pulses (80a) by the primary radiation source (12, 12a) primarily during the plurality of primary active periods (76; 76a).

13. The method according to any of the preceding claims, wherein the primary radiation (20, 20a) is detected by the primary detector (16; 16a) by direct conversion each time during the plurality of primary active periods (76; 76a).

14. A radiation system (10a; 10b) for handling radiation in relation to an object (22), the radiation system (10a; 10b) comprising:
- a primary radiation source (12, 12a) configured to emit primary radiation (20, 20a);
- a secondary radiation source (14; 14b) configured to emit secondary radiation (24; 24b);
- a primary detector (16; 16a) configured to detect the primary radiation (20, 20a) each time during a plurality of primary active periods (76; 76a), the primary radiation (20, 20a) having interacted with the object (22);
- a control system (18) comprising at least one data processing device (26) and at least one memory (28) having at least one computer program stored thereon, the at least one computer program comprising program code which, when executed by the at least one data processing device (26), causes the at least one data processing device (26) to:
- control the primary radiation source (12, 12a) to emit the primary radiation (20, 20a) onto the object (22);
- control the secondary radiation source (14; 14b) to emit the secondary radiation (24; 24b) onto the object (22) in secondary pulses (80; 80b) emitted during a plurality of primary inactive periods (78; 78a), interleaving each primary inactive period (78; 78a) with a primary active period (76; 76a);
- control the secondary radiation source (14; 14b) to not emit any secondary pulses (80; 80b) during the primary active periods (76; 76a);
and
- control the primary detector (16; 16a) to adopt an inactive state during each primary inactive period (78; 78a), where the primary detector (16; 16a) is inactive in relation to capturing incoming radiation during this primary inactive period (78; 78a).

15. The radiation system (10a; 10b) according to claim 14, wherein the at least one computer program comprises program code which, when executed by the at least one data processing device (26), causes the at least one data processing device (26) to:
- control the primary detector (16; 16a) to not detect incoming radiation during the primary inactive periods (78; 78a); and/or
- control the primary detector (16; 16a) to not collect detection data (58) indicative of incoming radiation during the primary inactive periods (78; 78a).

16. The radiation system (10a; 10b) according to claim 14 or 15, wherein the at least one computer program comprises program code which, when executed by the at least one data processing device (26), causes the at least one data processing device (26) to:
- control the primary detector (16; 16a) to collect detection data (58) indicative of the primary radiation (20, 20a) detected during the primary active periods (76; 76a).

17. The radiation system (10a; 10b) according to claim 16, wherein the primary detector (16; 16a) comprises a plurality of pixels (32), one or more counters (60) associated with each pixel (32), each configured to count the detection data (58) indicative of the primary radiation (20, 20a) detected during the primary active periods (76; 76a) to provide counted detection data (62), and a register (66) associated with each counter (60), each register (66) being configured to collect the counted detection data (62) from the associated counter (60).

18. The radiation system (10a; 10b) according to claim 17, wherein each register (66) is configured to collect the counted detection data (62) from the associated counter (60) in response to a collection signal (68), and wherein the at least one computer program comprises program code which, when executed by the at least one data processing device (26), causes the at least one data processing device (26) to command sending of the collection signal (68) to each register (66) during each primary inactive period (78; 78a).

19. The radiation system (10a; 10b) according to claim 17 or 18, wherein each counter (60) is configured to be reset in response to a reset signal (64), and wherein the at least one computer program comprises program code which, when executed by the at least one data processing device (26), causes the at least one data processing device (26) to command sending of the reset signal (64) to each counter (60) during each primary inactive period (78; 78a).

20. The radiation system (10a; 10b) according to any of claims 17 to 20, wherein each register (66) is configured to read out the counted detection data (62) in response to a readout signal (70), and wherein the at least one computer program comprises program code which, when executed by the at least one data processing device (26), causes the at least one data processing device (26) to command sending of the readout signal (70) to each register (66) during at least some of the primary inactive periods (78; 78a).

21. The radiation system (10a; 10b) according to any of claims 14 to 20, wherein the secondary radiation source (14; 14b) is a linear accelerator.

22. The radiation system (10a; 10b) according to any of claims 14 to 21, wherein a secondary pulse duration (82, 82b) of each secondary pulse (80; 80b) is less than 100 µs.

23. The radiation system (10a; 10b) according to any of claims 14 to 22, wherein a primary active period duration of each primary active period (76; 76a) is at least 1 ms.

24. The radiation system (10a; 10b) according to any of claims 14 to 23, wherein the secondary radiation (24; 24b) has energies of at least 0.5 MeV.

25. The radiation system (10a; 10b) according to any of claims 14 to 24, wherein the at least one computer program comprises program code which, when executed by the at least one data processing device (26), causes the at least one data processing device (26) to control the primary radiation source (12, 12a) to emit the primary radiation (20, 20a) onto the object (22) in primary pulses (80a) primarily during the plurality of primary active periods (76; 76a).

26. The radiation system (10a; 10b) according to any of claims 14 to 25, wherein the primary detector (16; 16a) is a direct conversion detector.
